# FASCICULE DE BREVET EUROPEEN

(11) **EP 0 975 313 B1**
(45) Date de publication et mention de la délivrance du brevet: **14.08.2002**
(21) Numéro de dépôt: 98963622.0
(22) Date de dépôt: 23.12.1998
(51) Int. Cl.: A61K 7/06

(54) **COMPOSITION COSMETIQUE A BASE DE POLYURETHANNES ASSOCIATIFS ET DE POLYMERES ANIONIQUES A CHAINES GRASSES**
KOSMETISCHE ZUSAMMENSETZUNG AUF DER BASIS VON ASSOZIATIVEN POLYURETHANEN UND ANIONISCHEN POLYMEREN MIT FETTKETTEN
COSMETIC COMPOSITION BASED ON ASSOCIATIVE POLYURETHANE AND ANIONIC POLYMERS WITH FATTY CHAINS

(30) Priorité: 13.02.1998 FR 9801774
(43) Date de publication de la demande: 02.02.2000
(73) Titulaire: L'OREAL, 75008 Paris (FR)
(72) Inventeur: DUPUIS, Christine, F-75018 Paris (FR)
(74) Mandataire: Miszputen, Laurent
(86) Numéro de dépôt international: FR9802863
(87) Numéro de publication internationale: WO9940890

(56) Documents cités:
- DE-A- 4 438 846
- FR-A- 2 733 910

## Description

La présente invention concerne des compositions cosmétiques contenant un nouveau système épaississant de milieux aqueux à base de polyuréthannes associatifs et de polymères anioniques à chaînes grasses, ainsi que leur utilisation en tant que gels coiffants ou gels de soin capillaires non rincés.

L'épaississement et/ou la gélification des milieux aqueux par des polymères est depuis longtemps un important de sujet de recherche cosmétique. L'obtention d'un effet d'épaississement intéressant par un polymère hydrosoluble suppose généralement une masse molaire élevée et un volume hydrodynamique important. La gélification d'un milieu aqueux est alors considérée comme le résultat d'un réseau polymère tridimensionnel obtenu par réticulation de polymères linéaires ou par copolymérisation de monomères bifonctionnels et polyfonctionnels. L'utilisation de tels polymères de masse molaire très élevée pose cependant un certain nombre de problèmes tels que la texture peu agréable et la difficulté d'étalement des gels obtenus.

Une approche intéressante a consisté à utiliser comme épaississants des polymères capables de s'associer réversiblement entre eux ou avec d'autres molécules ou particules. Cette association physique donne lieu à des systèmes macromoléculaires thixotropes ou rhéofluidifiables, c'est-à-dire des systèmes dont la viscosité dépend des forces de cisaillement auxquelles ils sont soumis.
De tels polymères capables de s'associer réversiblement entre eux ou avec d'autres molécules sont appelés "polymères associatifs". Les forces d'interactions en jeu peuvent être de nature très différente par exemple de nature électrostatique, de type liaisons hydrogène ou des interactions hydrophobes.
Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes par lesquelles les polymères interagissent et se rassemblent entre eux ou avec d'autres molécules.

Il est connu de préparer des compositions capillaires sous forme de gel utilisant, comme système épaississant, de tels polymères amphiphiles associatifs, en conjonction avec des agents tensioactifs. On pense que les propriétés rhéologiques intéressantes des gels ainsi obtenus sont dues à la formation de micelles mixtes contenant les agents tensioactifs et les parties hydrophobes des polymères amphiphiles, ces micelles constituant une multitude de points de réticulation physique.

Cependant, ces compositions à base de polymères associatifs et d'agents tensioactifs n'ont pas toujours les propriétés cosmétiques souhaitées. Ainsi, la présence d'agents tensioactifs, même en faibles quantités, peut modifier de façon indésirable les propriétés cosmétiques desdites compositions, telles que les propriétés d'application ou de toucher après séchage. Par ailleurs, notamment dans le domaine des gels de coiffage ou de soin non rincés, il est important de pouvoir répartir uniformément le produit sur l'ensemble de la chevelure de manière à éviter les surcharges et les défauts cosmétiques qui en résultent.

La demande de brevet européen EP-A-0 412 705 décrit des compositions cosmétiques, en particulier des compositions cosmétiques capillaires, utilisant comme système épaississant des polymères hydrosolubles non ioniques modifiés par introduction de chaînes grasses, en combinaison avec un ou plusieurs polymères hydrosolubles naturels ou synthétiques.

La demande de brevet français FR-A-2 733 910 divulgue des compositions pour mousses de coiffage contenant, en combinaison, au moins un polymère anionique et au moins un polyuréthanne associatif, l'un au moins de ces deux polymères ayant un pouvoir moussant, de manière à améliorer les propriétés des mousses obtenues,

On a découvert à présent qu'il était possible d'obtenir un bon effet épaississant, voire gélifiant, et des propriétés cosmétiques intéressantes en associant des polyuréthannes amphiphiles associatifs à des polymères anioniques comportant au moins un motif monomère à chaîne grasse.

Le gel obtenu par l'association de ces deux types de polymères a une texture très fondante et est agréable à appliquer. Le toucher final sur cheveux séchés est plus agréable et moins chargé, Le gel a par ailleurs un excellent pouvoir coiffant.

Un objet de la présente invention est donc une composition cosmétique comprenant au moins un polyuréthanne non ionique associatif en combinaison avec au moins un polymère anionique comportant au moins un motif monomère à chaîne grasse.

Un autre objet de la présente invention est l'utilisation de la combinaison d'au moins un polyuréthanne non ionique associatif et d'au moins un polymère anionique comportant au moins un motif monomère à chaîne grasse en tant que système épaississant pour des compositions cosmétiques.
Un troisième objet de l'invention est un procédé de traitement cosmétique des cheveux utilisant une composition cosmétique obtenue par association d'au moins un polyuréthanne associatif non ionique et d'au moins un polymère anionique comportant au moins un motif monomère à chaîne grasse.

D'autres objets apparaîtront à la lecture de la description et des exemples qui suivront.
Les compositions cosmétiques conformes à l'invention sont essentiellement caractérisées par le fait qu'elles contiennent, dans un milieu cosmétiquement acceptable,
(A) au moins un polyuréthanne associatif non ionique amphiphile correspondant à la formule générale 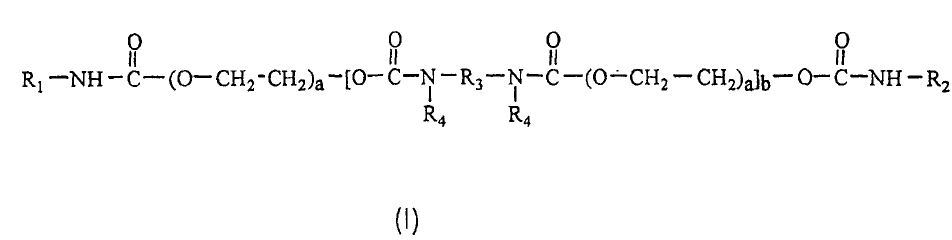 dans laquelle
   un des résidus R₁ et R₂ représente un groupe alkyle supérieur en C₈₋₁₈ et l'autre un groupe alkyle inférieur en C₁₋₆,
   R₃ représente un radical hydrocarboné en C₄₋₃₆, de préférence en C₆₋₁₀,
   R₄ représente un atome d'hydrogène ou un radical alkyle en C₁₋₆, de préférence un atome d'hydrogène,
   a varie indépendamment de 90 à 600, et
   b vaut de 1 à 4, et
(B) au moins un polymère anionique comportant au moins un motif monomère à chaîne grasse.

On entend par groupe alkyle inférieur en C₁₋₆, selon l'invention, un groupe alkyle à chaîne linéaire ou ramifiée comportant de 1 à 6 atomes de carbone, tel que les radicaux méthyle, éthyle, *n*-propyle, *n*-butyle, *n*-pentyle et *n*-hexyle ainsi que les isomères ramifiés correspondants.

Conformément à l'invention, les groupes alkyle supérieurs en C₈₋₁₈ désignent des groupes alkyle à chaîne linéaire ou ramifiée comportant de 8 à 18 atomes de carbone, tels que les radicaux octyle, nonyle, décyle, undécyle, dodécyle, tridécyle, tétradécyle, pentadécyle, hexadécyle, heptadécyle et octadécyle.

Dans un mode de réalisation préféré, un des radicaux alkyle R₁ et R₂ en a-W représente un groupe octadécyle et l'autre un groupe méthyle. Les polyuréthannes associatifs utilisés dans les compositions de la présente invention sont utilisés sous forme de solution ou suspension aqueuse contenant éventuellement une certaine quantité d'amidon soluble. Cet amidon peut être n'importe quel amidon extrait de sources naturelles, tel que l'amidon de blé, de maïs, de riz, de pomme de terre etc., et qui a été modifié par voie chimique, enzymatique ou microbiologique de manière à être soluble dans l'eau.

Un polymère préféré est commercialisé par la Société Rohm & Haas sous la dénomination ACRYSOL 46. Il s'agit d'un polyuréthanne obtenu par condensation d'hexaméthylènediisocyanate et de polyéthylèneglycol, et portant à ses extrémités respectivement en moyenne un résidu méthyle et un résidu octadécyle. Ce polymère se présente sous forme d'une solution aqueuse à 15 % en poids de matière active polyuréthanne contenant, en plus, de 3 - 5 % d'une matrice d'amidon modifié par voie enzymatique.

Les polymères anioniques à chaînes grasses de la présente invention constituant le composant (B) sont en particulier des polymères comportant des motifs dérivés d'acides carboxyliques, d'acides phosphoniques ou d'acides sulfoniques et au moins un motif portant une chaîne grasse.

Les groupements anioniques sont choisis par exemple parmi les groupements dérivés d'acides carboxyliques tels que l'acide acrylique, l'acide méthacrylique, l'acide crotonique, l'acide maléique, l'acide fumarique, l'acide itaconique, d'acides sulfoniques tels que l'acide vinylsulfonique, l'acide styrènesulfonique, ou d'acides phosphoniques tels que l'acide vinylphosphonique ou styrènephosphonique.

Les polymères anioniques à chaînes grasses de la présente invention peuvent également contenir un ou plusieurs motifs non ioniques bien connus dans la technique, par exemple des motifs dérivés de monomères vinyliques, oléfiniques, styréniques, acryliques ou méthacryliques. On peut citer à titre d'exemple de tels monomères, l'éthylène, le propylène, le styrène, l'acétate de vinyle, les acrylates et méthacrylates d'alkyle.
Les chaînes grasses sont des groupements alkyle en C₈₋₂₂, linéaires ou ramifiés. Elles peuvent être dérivées de monomères tels que les acrylates ou méthacrylates d'alkyle en C₈₋₂₂ ou les esters vinyliques d'acides gras supérieurs en C₈₋₂₂.

Les polymères anioniques à chaînes grasses de la présente invention peuvent être préparés par copolymèrisation de monomères anioniques et de monomères comportant au moins une chaîne grasse et, éventuellement, de monomères non ioniques. Ces monomères non ioniques peuvent être vinyliques, oléfiniques, styrèniques, acryliques ou méthacryliques. On peut également envisager de les préparer en introduisant les groupements anioniques et les chaînes grasses par greffage ou modification chimique de polymères naturels ou synthétiques

On peut citer comme exemples de polymères anioniques préférés de la présente invention, les terpolymères d'acide acrylique, de vinylpyrrolidone et de méthacrylate d'alkyle en C₈₋₁₈, par exemple de lauryle, tels que celui commercialisé sous la dénomination ACRYLIDONE LM par la société ISP; les terpolymères d'acétate de vinyle, de maléate de monoisobutyle et d'un alcanoate de vinyle en C₁₀₋₂₀, par exemple de néodécanoate de vinyle, tels que celui commercialisé sous la dénomination MEYPRO-FIX 509 par la société Rhône Poulenc Surfactants; et les terpolymères d'acétate de vinyle, d'acide crotonique et d'un alcanoate de vinyle en C₁₀₋₂₀, par exemple de néodécanoate de vinyle, tels que celui commercialisé sous la dénomination NATIONAL 28-2930 par la société National Starch.

Selon l'invention, les polyuréthannes associatifs et les polymères à chaînes grasses sont utilisés en des quantités suffisantes pour obtenir un épaississement ou une gélification satisfaisante du milieu aqueux.

On recommande notamment une quantité de polyuréthannes associatifs comprise entre 0,1 et 10 % en poids et, de préférence, entre 0,5 et 5 % en poids exprimé en matière active et rapportée au poids total de la composition.
Dans les compositions de la présente invention, les polymères anioniques comportant au moins une chaîne grasse sont présents à raison de 0,01 à 10 % en poids, de préférence à raison de 0,1 à 5 % en poids de matière active par rapport au poids total de la composition.
Dans la présente invention , le rapport dudit polyuréthane associatif non ionique (A) de formule (I) audit polymère anionique comportant au moins un motif monomère à chaîne grasse (B) est compris de préférence dans l'intervalle allant de 90/10 à 10/90.

Le milieu cosmétiquement acceptable est constitué de préférence d'eau et peut contenir en outre des solvants cosmétiquement acceptables, par exemple des monoalcools inférieurs tels que l'éthanol ou l'isopropanol, des glycols tels que le diéthylèneglycol, des éthers de glycols tels que les alkyl-éthers d'éthylèneglycol ou de diéthylèneglycol, ou encore des esters d'acides gras, tous ces solvants étant utilisés seuls ou sous forme de mélange.

Les gels coiffants ou de soins peuvent contenir en outre un ou plusieurs additifs utilisés habituellement dans de telles compositions capillaires. On peut citer à titre d'exemple les parfums, colorants, conservateurs, filtres solaires, vitamines, agents régulateurs de pH etc. Il est bien entendu que le choix de ces composés doit tenir compte d'éventuelles interactions avec le système épaississant. L'homme du métier veillera à ce que l'adjonction de ces additifs n'aura pas d'influence défavorable sur les propriétés avantageuses des compositions obtenues grâce à la présente invention.

Un procédé de traitement cosmétique des cheveux préféré, selon l'invention, consiste à appliquer et à répartir de façon homogène les compositions décrites ci-dessus sur les cheveux et à sécher les cheveux ainsi traités sans les rincer.

Les exemples suivants sont destinés à illustrer l'invention sans pour autant présenter un caractère limitatif.

### Exemple 1

On prépare les compositions aqueuses suivantes :

L'ACRYSOL 46, produit commercialisé par la société Rohm et Haas, est un polyuréthanne obtenu par condensation d'hexaméthylènediisocyanate et de polyéthylèneglycol, et portant à ses extrémités respectivement en moyenne un résidu méthyle et un résidu octadécyle. La résine NATIONAL 28-2930, commercialisée par la société National Starch, est un terpolymère anionique obtenu par copolymérisation d'acétate de vinyle, d'acide crotonique et de néodécanoate de vinyle. Cet exemple montre que l'association du polyuréthanne associatif préféré de la présente invention (ACRYSOL 46) et d'un polymère anionique à chaînes grasses (NATIONAL 28 2930) permet d'obtenir un gel ayant d'excellentes propriétés cosmétiques. Les cheveux traités avec cette composition A sont faciles à démêler, souples et lisses au toucher.
On peut noter que l'ACRYSOL 46 *seul* (composition B) ne conduit à aucun effet épaississant notable et encore moins à une gélification.
La composition A présente également l'avantage d'être fondante et non grasse et de ne pas poisser.

### Exemple 2

On a préparé un gel de soin ayant la composition suivante :

| | |
|---|---|
| ACRYSOL 46 | 2 % de matière active |
| ACRYLIDONE LM* | 1 % de matière active |
| 2-amino-2-méthyl-1-propanol | q.s. p. neutralisation |
| parfum, colorant, conservateur et eau déminéralisée | q. s. p. 100 g |

| | |
|---|---|
| * L'ACRYLIDONE LM est un terpolymère anionique obtenu par copolymérisation d'acide acrylique, de vinylpyrrolidone et de méthacrylate de lauryle (68/23/9 %), commercialisé par la société I.S.P. | |

### Exemple 3

On a préparé un gel de soin ayant la composition suivante :

| | |
|---|---|
| ACRYSOL 46 | 2 % de matière active |
| ACRYLIDONE LM | 2 % de matière active |
| 2-amino-2-méthyl-1-propanol | q.s. p. neutralisation |
| parfum, colorant, conservateur et eau déminéralisée | q. s. p. 100 g |

### Exemple 4

On a préparé un gel de soin ayant la composition suivante :

| | |
|---|---|
| ACRYSOL 46 | 3 % de matière active |
| MEYPRO-FIX 509* | 2 % de matière active |
| 2-amino-2-méthyl-1-propanol | q.s. p. neutralisation |
| parfum, colorant, conservateur et eau déminéralisée | q. s. p. 100 g |

| | |
|---|---|
| *Le MEYPRO-FIX 509 est un terpolymère anionique d'acétate de vinyle, de maléate de monoisobutyle et de néodécanoate de vinyle, commercialisé par la société Rhône Poulenc Surfactants. | |

Il est bien entendu que la description qui précède n'a été donnée qu'à titre purement illustratif et non limitatif et que des variantes ou des modifications peuvent y être apportées dans le cadre de la présente invention.

## Revendications

1. Composition cosmétique **caractérisée par le fait qu'**elle comprend, dans un milieu cosmétiquement acceptable,
(A) au moins un polyuréthanne associatif non ionique amphiphile correspondant à la formule générale 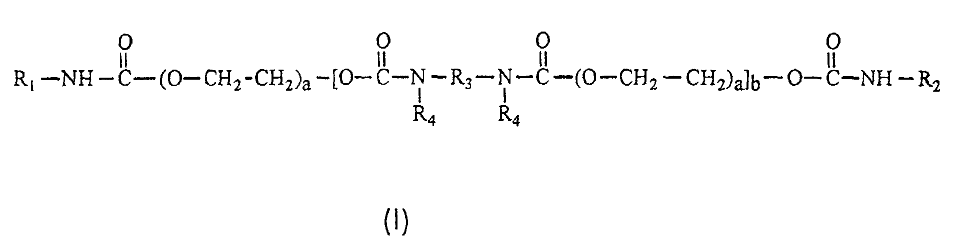 dans laquelle
un des résidus R₁ et R₂ représente un groupe alkyle supérieur en C₈₋
₁₈ et l'autre un groupe alkyle inférieur en C₁₋₆,
R₃ représente un radical hydrocarboné en C₄₋₃₆, de préférence en C₆₋₁₀,
R₄ représente un atome d'hydrogène ou un radical alkyle en C₁₋₆, de préférence un atome d'hydrogène,
a varie indépendamment de 90 à 600, et
b vaut de 1 à 4, et
(B) au moins un polymère anionique comportant au moins un motif dérivé d'un monomère à chaîne grasse.

2. Composition cosmétique selon la revendication 1, **caractérisée en ce que** le composant (A) est un polyuréthanne associatif non ionique dans lequel en moyenne un des radicaux R₁ et R₂ en a-W représente un groupe octadécyle et l'autre un groupe méthyle.

3. Composition cosmétique selon la revendication 1 ou 2, **caractérisée par le fait que** le composant (A) se présente sous forme d'une solution ou suspension dans l'eau contenant également de l'amidon soluble modifié par voie chimique, enzymatique ou microbiologique.

4. Composition cosmétique selon l'une quelconque des revendications 1 ou 2, **caractérisée en ce que** les polymères constituant le composant (B) comportent des motifs dérivés d'acides carboxyliques, d'acides phosphoniques ou d'acides sulfoniques et au moins un motif portant une chaîne grasse.

5. Composition selon la revendication 4, **caractérisée en ce que** les groupements anioniques sont choisis parmi les groupements dérivés d'acides carboxyliques tels que l'acide acrylique, l'acide méthacrylique, l'acide crotonique, l'acide maléique, l'acide fumarique, l'acide itaconique, les groupements dérivés d'acides sulfoniques tels que l'acide vinylsulfonique, l'acide styrènesulfonique, et les groupements dérivés d'acides phosphoniques tels que l'acide vinylphosphonique ou styrènephosphonique.

6. Composition selon l'une quelconque des revendications 1 à 5 **caractérisée en ce que** les motifs comportant une chaîne grasse sont dérivés de monomères comportant au moins une chaîne alkyle en C₈₋₂₂, linéaire ou ramifiée.

7. Composition selon la revendication 6 **caractérisée par le fait que** ledit monomère portant au moins une chaîne alkyle est choisi parmi les acrylates ou méthacrylates d'alkyle en C₈₋₂₂ ou les esters vinyliques d'acides gras supérieurs en C₈₋₂₂.

8. Composition selon l'une quelconque des revendications 1 à 7, **caractérisée en ce que** lesdits polymères anioniques à chaînes grasses contiennent également des motifs non ioniques.

9. Composition selon la revendication 8, **caractérisée en ce que** lesdits motifs non ioniques sont dérivés de monomères vinyliques, oléfiniques, styréniques, acryliques ou méthacryliques.

10. Composition selon l'une quelconque des revendications 1 à 9, **caractérisée en ce qu'**elle contient le composant (A) à raison de 0,1 à 10 % en poids et, de préférence de 0,5 à 5 % en poids, exprimé en matière active rapportée au poids total de la composition.

11. Composition selon l'une quelconque des revendications 1 à 10, **caractérisée en ce qu'**elle contient le composant (B) à raison de 0,01 à 10 % en poids, de préférence à raison de 0,1 à 5 % en poids de matière active par rapport au poids total de la composition.

12. Composition selon l'une quelconque des revendications 1 à 11 **caractérisée par le fait que** le rapport en poids dudit polyuréthanne associatif non ionique de formule (I) audit polymère anionique comportant au moins un motif monomère à chaîne grasse est compris dans l'intervalle allant de 90/10 à 10/90.

13. Composition selon l'une quelconque des revendications 1 à 12, **caractérisée par le fait qu'**elle se présente sous forme d'un gel de coiffage ou d'un gel de soin capillaire non rincé.

14. Utilisation de l'association d'un polyuréthanne associatif non ionique de formule (I) et d'un polymère anionique comportant au moins une chaîne grasse, en tant que système épaississant d'une composition cosmétique.

15. Procédé de traitement cosmétique des cheveux, **caractérisé par le fait que** l'on applique sur les cheveux la composition définie selon l'une quelconque des revendications 1 à 13 et en ce que l'on sèche les cheveux ainsi traités sans les rincer.

## Patentansprüche

1. Kosmetische Zusammensetzung, **dadurch gekennzeichnet, dass** sie in einem kosmetisch akzeptablen Medium die folgenden Komponenten enthält:
(A) mindestens ein amphiphiles nichtionisches assoziatives Polyurethan der folgenden allgemeinen Formel: 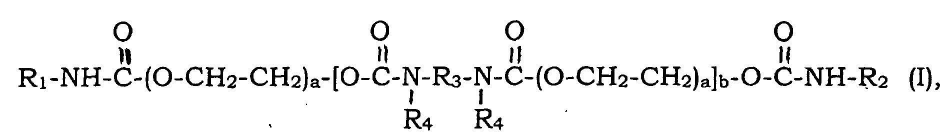 worin bedeuten:
- einer der Reste R₁ und R₂ eine höhere C₈₋₁₈-Alkylgruppe und der andere Rest eine niedere C₁₋₆-Alkylgruppe,
- R₃ eine Kohlenwasserstoffgruppe mit 4 bis 36 Kohlenstoffatomen und vorzugsweise mit 6 bis 10 Kohlenstoffatomen,
- R₄ Wasserstoff oder eine C₁₋₆-Alkylgruppe und vorzugsweise Wasserstoff,
- die Indices a unabhängig voneinander Werte im Bereich von 90 bis 600 und
- b 1 bis 4,
und
(B) mindestens ein anionisches Polymer, das mindestens eine Einheit aufweist, die von einem Monomer mit Fettkette abgeleitet ist.

2. Kosmetische Zusammensetzung nach Anspruch 1, **dadurch gekennzeichnet, dass** die Komponente (A) ein nichtionisches assoziatives Polyurethan ist, worin im Mittel eine der Gruppen R₁ und R₂ in α-ω-Stellung eine Octadecylgruppe und die andere Gruppe eine Methylgruppe bedeutet.

3. Kosmetische Zusammensetzung nach Anspruch 1 oder 2, **dadurch gekennzeichnet, dass** die Komponente (A) in Form einer Lösung oder Suspension in Wasser vorliegt, die ferner auf chemischem, enzymatischem oder mikrobiologischem Wege modifizierte lösliche Stärke enthält.

4. Kosmetische Zusammensetzung nach einem der Ansprüche 1 oder 2, **dadurch gekennzeichnet, dass** die Polymere, die die Komponente (B) bilden, von Carbonsäuren, Phosphonsäuren oder Sulfonsäuren abgeleitete Einheiten und mindestens eine Einheit, die eine Fettkette trägt, aufweisen.

5. Zusammensetzung nach Anspruch 4, **dadurch gekennzeichnet, dass** die anionischen Gruppen unter Gruppen, die von Carbonsäuren abgeleitet sind, wie Acrylsäure, Methacrylsäure, Crotonsäure, Maleinsäure, Fumarsäure und Itaconsäure, Gruppen, die von Sulfonsäuren abgeleitet sind, wie Vinylsulfonsäure und Styrolsulfonsäure, und Gruppen, die von Phosphonsäuren abgeleitet sind, wie Vinylphosphonsäure oder Styrolphosphonsäure, ausgewählt sind.

6. Zusammensetzung nach einem der Ansprüche 1 bis 5, **dadurch gekennzeichnet, dass** die Einheiten, die eine Fettkette tragen, von Monomeren abgeleitet sind, die mindestens eine lineare oder verzweigte C₈₋₂₂-Alkylkette aufweisen.

7. Zusammensetzung nach Anspruch 6, **dadurch gekennzeichnet, dass** das Monomer, das mindestens eine Alkylkette aufweist, unter C₈₋₂₂-Alkylacrylaten, C₈₋₂₂-Alkylmethacrylaten oder Vinylestern von höheren Fettsäuren mit 8 bis 22 Kohlenstoffatomen ausgewählt ist.

8. Zusammensetzung nach einem der Ansprüche 1 bis 7, **dadurch gekennzeichnet, dass** die anionischen Polymere mit Fettketten ferner nichtionische Einheiten enthalten.

9. Zusammensetzung nach Anspruch 8, **dadurch gekennzeichnet, dass** die nichtionischen Einheiten von Vinylmonomeren, Olefinmonomeren, Styrolmonomeren, Acrylmonomeren oder Methacrylmonomeren abgeleitet sind.

10. Zusammensetzung nach einem der Ansprüche 1 bis 9, **dadurch gekennzeichnet, dass** sie die Komponente (A) in einem als Wirkstoff ausgedrückten Mengenanteil von 0,1 bis 10 Gew.-% und vorzugsweise von 0,5 bis 5 Gew.-%, bezogen auf das Gesamtgewicht der Zusammensetzung, enthält.

11. Zusammensetzung nach einem der Ansprüche 1 bis 10, **dadurch gekennzeichnet, dass** sie die Komponente (B) in einem als Wirkstoff ausgedrückten Mengenanteil von 0,01 bis 10 Gew.-% und vorzugsweise von 0,1 bis 5 Gew.-%, bezogen auf das Gesamtgewicht der Zusammensetzung, enthält.

12. Zusammensetzung nach einem der Ansprüche 1 bis 11, **dadurch gekennzeichnet, dass** das Gewichtsverhältnis des nichtionischen assoziativen Polyurethans der Formel (I) zu dem anionischen Polymer, das mindestens eine Monomereinheit mit Fettkette aufweist, im Bereich von 90/10 bis 10/90 liegt.

13. Zusammensetzung nach einem der Ansprüche 1 bis 12, **dadurch gekennzeichnet, dass** sie in Form eines Frisiergels oder eines Haarpflegegels vorliegt, das nicht ausgespült wird.

14. Verwendung der Kombination eines nichtionischen assoziativen Polyurethans der Formel (I) und eines anionischen Polymers, das mindestens eine Fettkette aufweist, als Verdickungssystem für eine kosmetische Zusammensetzung.

15. Verfahren zur kosmetischen Behandlung der Haare, **dadurch gekennzeichnet, dass** die in einem der Ansprüche 1 bis 13 definierte Zusammensetzung auf die Haare aufgebracht wird und dadurch, dass die so behandelten Haare ohne Spülen getrocknet werden.

## Claims

1. Cosmetic composition, **characterized in that** it comprises, in a cosmetically acceptable medium,
(A) at least one amphiphilic nonionic associative polyurethane corresponding to the general formula 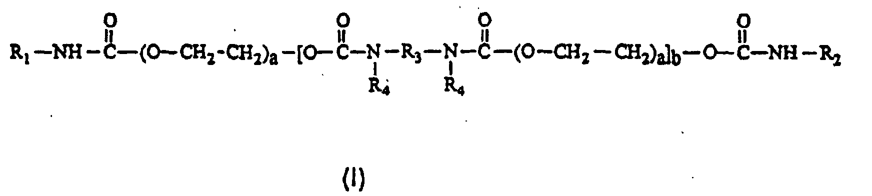 in which
one of the residues R₁ and R₂ represents a higher C₈-C₁₈. alkyl group and the other represents alower C₁-C₆ alkyl group,
R₃ represents a C₄-C₃₆, preferably C₆-C₁₀, hydrocarbon-based radical,
R₄ represents a hydrogen atom or a C₁-C₆ alkyl radical, preferably a hydrogen atom,
a ranges, independently, from 90 to 600, and
b is from 1 to 4, and
(B) at least one anionic polymer comprising at least one unit derived from a fatty-chain monomer.

2. Cosmetic composition according to Claim 1, **characterized in that** the component (A) is a nonionic associative polyurethane in which, on average, one of the radicals R₁ and R₂ in an α-ω position represents an octadecyl group and the other represents a methyl group.

3. Cosmetic composition according to Claim 1 or 2, **characterized in that** the component (A) is in the form of a solution or suspension in water also containing chemically, enzymatically or microbiologically modified soluble starch.

4. Cosmetic composition according to either of Claims 1 and 2, **characterized in that** the polymers constituting the component (B) comprise units derived from carboxylic acids, from phosphonic acids or from sulphonic acids, and at least one unit bearing a fatty chain.

5. Composition according to Claim 4, **characterized in that** the anionic groups are chosen from groups derived from carboxylic acids, such as acrylic acid, methacrylic acid, crotonic acid, maleic acid, fumaric acid or itaconic acid, groups derived from sulphonic acids, such as vinylsulphonic acid or styrenesulphonic acid, and groups derived from phosphonic acids, such as vinylphosphonic acid or styrenephosphonic acid.

6. Composition according to any one of Claims 1 to 5, **characterized in that** the units comprising a fatty chain are derived from monomers comprising at least one linear or branched C₈-C₂₂ alkyl chain.

7. Composition according to Claim 6, **characterized in that** the said monomer bearing at least one alkyl chain is chosen from C₈-C₂₂ alkyl acrylates or methacrylates or vinyl esters of higher C₈-C₂₂ fatty acids.

8. Composition according to any one of Claims 1 to 7, **characterized in that** the said fatty-chain anionic polymers also contain nonionic units.

9. Composition according to Claim 8, **characterized in that** the said nonionic units are derived from vinyl, olefinic, styrene, acrylic or methacrylic monomers.

10. Composition according to any one of Claims 1 to 9, **characterized in that** it contains the component (A) in a proportion of from 0.1 to 1,0% by weight, and preferably from 0.5 to 5% by weight, expressed as active material relative to the total weight of the composition.

11. Composition according to any one of Claims 1 to 10, **characterized in that** it contains the component (B) in a proportion of from 0.01 to 10% by weight, preferably in a proportion of from 0.1 to 5% by weight, of active material relative to the total weight of the composition.

12. Composition according to any one of Claims 1 to 11, **characterized in that** the weight ratio of the said nonionic associative polyurethane of formwla (I) to the said anionic polymer comprising at least one fatty-chain monomer unit is within the range from 90/10 to 10/90.

13. Composition according to any one of Claims 1 to 12, **characterized in that** it is in the form of a leave- in haircare gel or styling gel.

14. Use of the combination of a nonionic associative polyurethane of formula (I) and an anionic polymer comprising at least one fatty chain, as a system for thickening a cosmetic composition.

15. Cosmetic process for treating the hair, **characterized in that** the composition defined according to any one of Claims 1 to 13 is applied to the hair and the hair thus treated is dried without rinsing it.
